(19) Europäisches Patentamt
European Patent Office
Office européen des brevets

(11) **EP 2 897 709 B1**

(12) **EUROPEAN PATENT SPECIFICATION**

(45) Date of publication and mention
of the grant of the patent:
**30.10.2019 Bulletin 2019/44**

(51) Int Cl.:
**B01D 43/00** *(2006.01)*    **G01N 29/00** *(2006.01)*
**G01N 33/50** *(2006.01)*

(21) Application number: **13839064.6**

(22) Date of filing: **20.09.2013**

(86) International application number:
**PCT/SE2013/051101**

(87) International publication number:
**WO 2014/046605 (27.03.2014 Gazette 2014/13)**

(54) **A METHOD FOR SEPARATING CELLS-BEAD COMPLEXES**

VERFAHREN ZUR TRENNUNG VON ZELLKÜGELCHENKOMPLEXEN

PROCÉDÉ DE SÉPARATION DE COMPLEXES CELLULES-BILLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.09.2012 SE 1251064
01.10.2012 US 201261708169 P**

(43) Date of publication of application:
**29.07.2015 Bulletin 2015/31**

(73) Proprietor: **AcouSort AB
223 81 Lund (SE)**

(72) Inventors:
• **AUGUSTSSON, Per
227 31 Lund (SE)**
• **LENSHOF, Andreas
226 47 Lund (SE)**
• **LAURELL, Thomas
224 67 Lund (SE)**
• **SCHEDING, Stefan
222 41 Lund (SE)**

(74) Representative: **Brann AB
P.O. Box 3690
Drottninggatan 27
103 59 Stockholm (SE)**

(56) References cited:
**WO-A1-2013/048323    WO-A1-2013/048323**

WO-A2-03/102737    WO-A2-2007/128795
WO-A2-2012/135663    US-A1- 2009 042 310

• **PER AUGUSTSSON ET AL: "Decomplexing
biofluids using microchip based
acoustophoresis", LAB ON A CHIP, vol. 9, no. 6,
1 January 2009 (2009-01-01) , pages 810-818,
XP055183122, ISSN: 1473-0197, DOI:
10.1039/B811027A**
• **AUGUSTSSON, P. ET AL.: 'Microfluidic,
Label-Free Enrichment of Prostate Cancer Cells
in Blood Based on Acoustophoresis'
ANALYTICAL CHEMISTRY vol. 84, 2012, pages
7954 - 7962, XP055193403**
• **MANNEBERG O. ET AL.: 'Wedge transducer
design for two-dimensional ultrasonic
manipulation in a microfluidic chip' JOURNAL OF
MICROMECHANICS AND MICROENGINEERING
vol. 18, no. 095025, 2008, pages 1 - 9,
XP020145127**
• **MANNEBERG O. ET AL.: 'Spatial confinement of
ultrasonic force filds in microfluidic chanels'
ULTRASONICS vol. 49, 2009, pages 112 - 119,
XP025715113**
• **LAURELL T. ET AL.: 'Chip integrated strategies
for acoustic separation and manipulation of cells
and particles' CHEMICAL SOCIETY REVIEWS vol.
36, 2007, pages 492 - 506, XP008117651**
• **PETERSON F. ET AL.: 'Free flow
acoustophoresis: Microfluidic-based mode of
particle and cell separation' ANALYTICAL
CHEMISTRY vol. 79, no. 14, 2007, pages 5117 -
5123, XP002589704**

## Description

FIELD OF THE INVENTION

This disclosure relates to a method and system to separate a subgroup of cells from a mixture of cells. This is achieved by creating a complex consisting of the subgroup of cells that are to be separated and a ligand that could be constructed of molecules such as antibodies, monoclonal antibodies, aptamers, other antigen specific molecules or fragments thereof specific for said subgroup of cells and microbeads present in a suspension. The suspension is then exposed to an acoustic field in one or two dimensions, which forces said cells and cell-bead complexes to separate from each other when migrating across the interface between two properly chosen liquids which have a difference in density or compressibility.

BACKGROUND

Affinity specific separations using microbeads in microfluidics system have emerged as a powerful means of targeted extraction of biological components in crude samples. This has since long been a standard in chromatography based systems, with packed microbead columns, for purification/enrichment of molecular species. Extraction of microbial and cellular targets from crude suspensions, however, have had limited success in packed bed based systems. A breakthrough for cell based separation along this line was the introduction of magnetic microbeads/nanobeads activated with antibodies against cell specific epitopes. In 1977 Molday et al. published a seminal paper where anti-mouse immunoglobuling activated magnetic nanoparticles (diameter□≈ 40 nm) bound to B-cells. The microbead coated B-cells were subsequently retained in a magnetic field generated by a horseshoe magnet while unlabeled cells were washed away [R. S. Molday, S. P. S. Yen and A. Rembaum, Nature, 1977, 268, 437-438.]. Improved extraction efficiency and process speed was obtained by passing the labeled cell solution past a dense network of ferromagnetic steel wire with the external magnet applied [R. S. Molday and L. L. Molday, Febs Letters, 1984, 170, 232-238.]. This provided locally stronger gradient field in the wire mesh and thus a higher retaining force on the labeled cells were obtained. This concept was further developed by Miltenyi [S. Miltenyi, W. Muller, W. Weichel and A. Radbruch, Cytometry, 1990, 11, 231-238.] and was commercialized by Miltenyi Biotech GmbH, providing new methods for affinity specific cell separation in preclinical and clinical preparatory applications of cell sorting and cell based therapy.

A further manifestation of such cell specific isolation is test-tube based magnetic affinity-bead extraction. A suspension containing cells is first incubated with microbeads coated with antibodies so that cell-bead complexes can form involving those cells that present on their surface the target molecules of the antibodies. Thereafter a magnet is placed close to the vessel containing the suspension whereby cell-bead complexes migrate towards the wall. The supernatant liquid is removed bringing along the non-targeted cells. The magnet is thereafter removed and the cell-bead complexes are resuspended in new cell medium, (www.invitrogen.com/dynal) Isolation of cells based on magnetic forces has two major drawbacks.

(1) The separation is strictly binary in the sense that if one or more beads are present on a cell, the cell will be captured by the magnetic field. Selective isolation of cells based on their expression level of certain surface molecule is therefore not feasible with current methods since they are not sensitive to the bead-load of the cells.
(2) Magnetic separation is well suited for batch processing wherein marked cells are trapped and wherein the trapping function has a limiting capacity. Continuous processing is difficult to achieve with magnetic forces since the magnetic beads will become trapped on the channel walls closest to the external magnetic field which causes a buildup of material in the flow channel that hinders the flow and prevents the targeted cell-bead complexes to be transferred.

Acoustophoresis, for which acoustically induced forces are utilized, provides a continuous process for the separation which is not limited by the volume of the sample. WO2007128795 discloses a method where an element to be separated from a fluid binds to affinity bearing particles and is to be moved by an ultrasonic wave field into a center flow of pure fluid. So far affinity-bead assisted extraction of cells has not been successful in acoustic separation systems. We propose herein that when introducing a second, properly chosen, liquid of different physical properties than the initial suspending liquid the discrimination of non-targeted cells increases vastly.

SUMMARY OF THE INVENTION

The herein described method based on acoustic forces, acoustophoresis, circumvent the abovementioned drawbacks of magnetic extraction of cell-bead complexes from non-complexed cells by discrimination objects in liquid suspensions based on size, shape, density and compressibility in a miniaturized continuous flow format. Acoustophoresis has proven gentle to cells and does not affect cell viability or cell function. Previously acoustophoresis has been demonstrated for label-free sorting of cells into discrete fractions based on their individual physical properties. This disclosure relates to a novel manifestation of acoustophoresis where the bead-cell complexes are transferred from one suspending liquid into another, which has been modified to have different physical properties. The differences in properties between the two liquids causes the cells that are not complexed with beads to be immobilized in the interface between the liquids while bead-complexed cells continue further into the modified liquid. The number of beads, which reflects the expression level of the targeted surface molecule, dictates how far into the modified liquid the cell-bead complex will travel.

Acoustic standing wave force fields have been used extensively to focus/concentrate cells in microfluidic system, utilizing the fact that the flow channel serves as the fluid conduit at the same time as it acts as an acoustic resonator defined by the channel dimension. Efforts to separate different cells in acoustic force-fields based on their intrinsic acoustophysical parameters have been reported but have still suffered from the parabolic flow profile causing a variation in retention time in the force field performing the separation step.

The current disclosure discloses how a suspension of different groups of cells, such as white blood cells are mixed with a ligand such as an antibody/monoclonal antibody or fragment thereof and microbeads, wherein said ligand binds to a specific subgroup of cells, and the microbeads binds to said ligand forming cell-bead complexes. Pre-alignment of the cells and the cell-bead complexes may then be performed by designing a channel such that an acoustic standing wave field exerts forces on cells and cell-bead complexes in two dimensions. Pre-aligned cells and cell-bead complexes subsequently enter into a microchannel segment designed for an acoustic standing wave resonance mode, which yields a force field that performs the separation of the non-complexed cell and the cell-bead complexes along a single dimension. The pre-alignment of cells and cell-bead complexes ensures equal retention time in the separation zone, and hence problems commonly experienced in poor separation resolution due to the parabolic flow profile of laminar flow conditions are alleviated. Crucial for this invention is that during the separation, the cell-bead complexes migrate from one suspending liquid into cell/bead-free **acoustic step gradient liquid (ASGL)**. Non-complexed cells are retained in the interface between the liquids due to their low migration rate in the ASGL. Microbeads have different physical properties, which mediates a higher migration rate in the ASGL and therefore the cell-bead complexes will migrate further into this liquid.

In one aspect the invention relates to a micro scale method for separating cell-bead complexes, the cell bead complexes comprising cells, ligands and beads, from a mixture of different types of cells in a liquid suspension, the suspension further comprising a suspending liquid, comprising the steps of;

i) subjecting the suspension to pressure, wherein said pressure forces said suspension into at least one inlet (11) and into at least one channel present on a microfluidic chip,
ii) forcing said suspension through the side branches of a trifurcated inlet while forcing cell free acoustic step gradient liquid, the acoustic step gradient liquid having physical properties which are different from the physical properties of the suspending liquid, through the central branch of said trifurcated inlet (12) simultaneously into at least one separation channel, wherein said suspension is pushed by hydrodynamic forces towards the walls of the separation channel,

iii) subjecting said mixture of different cells and said bead-cell complexes to a one dimensional acoustic force directed perpendicular to the length direction towards the center of the separation channel (16) and

iv) collecting the cell-bead complexes at the end of the separation channel present on the microfluidic chip through at least one first outlet (13),
vi) collecting said mixture of different types of cells at the end of the separation channel present on the microfluidic chip through at least one second outlet (14).

For the first time it is possible to separate cells having the same or similar characteristics by combining affinity specific coupling technology with the acoustic microfluidic technology and thereby be able to separate for example T-cells or from a population of white blood cells. This is achieved by forming complexes between specific subgroups of cells with ligands and microbeads and by introducing a two-dimensional acoustic force field in at least one pre-alignment microchannel where cells/particles can be directed into a defined position in the y-z-plane. This confines cell-bead complexes and cells to one or more discrete positions with a uniform flow velocity in the plane transverse to the flow, prior to entering the separation channel, hence making the separation deterministic such that the intrinsic properties of the cells and the fluid dynamic interaction between complexes and cells and the aqueous medium governs the separation outcome. The combined effect of pre-alignment and subsequent ASGL separation will thus increase the resolving power of such systems and enables for the first time the possibility to separate cell-bead complexes from other cells in a manner which is gentle, quantitative and which is well suited for upstream preconditioning and downstream analysis and treatment of acquired cells.

BRIEF DESCRIPTION OF THE DRAWINGS

Fig. 1. Schematic of on-chip transport of two suspended species. Top view. Not to scale.

Fig. 2. Schematic of on-chip transport of two suspended species. Side cross-sectional view. Not to scale.

Fig. 3. Drawing suggested realization of the complete device. Top view. To scale.

Fig. 4. Drawing suggested realization of the complete device. Side cross sectional view as if cut in half along the vertical center plane. To scale.

Fig. 5. Schematic of the external fluidics driving the flows in the channel.

Fig. 6. Schematic of the ASGL based separation principle

DETAILED DESCRIPTION OF THE INVENTION

**Definitions**

Letters (x), (y) and (z) refers to the spatial position along the length (l), width (w), and the height (h) of the microchannel, respectively. Letters $(Q_i)$, (v), and $(p_i)$ refers to volume flow rate, flow velocity and pressure, respectively where subscript (i) indicate multiple instances of a property.
The word suspension refers to a fluid containing solid particles or cells that are sufficiently large for sedimentation.
"Antibodies" as used herein includes polyclonal and monoclonal antibodies, chimeric, single chain, and humanized antibodies, as well as Fab fragments, including the products of a Fab or other immunoglobulin expression library. Antibodies, which consist essentially of pooled monoclonal antibodies with different epitopic specificities, as well as distinct monoclonal antibody preparations, are provided. Monoclonal antibodies are made by methods well known to those skilled in the art. The term antibody as used in this disclosure is meant to include intact molecules as well as fragments thereof, such as Fab and F(ab').sub.2, Fv and SCA fragments which are capable of binding an epitopic determinant on a protein of interest. A Fab fragment consists of a mono-valent antigen-binding fragment of an antibody molecule, and can be produced by digestion of a whole antibody molecule with the enzyme papain, to yield a fragment consisting of an intact light chain and a portion of a heavy chain. A Fab' fragment of an antibody molecule can be obtained by treating a whole antibody molecule with pepsin, followed by reduction, to yield a molecule consisting of an intact light chain and a portion of a heavy chain. Two Fab' fragments are obtained per antibody molecule treated in this manner. An (Fab').sub.2 fragment of an antibody can be obtained by treating a whole antibody molecule with the enzyme pepsin, without subsequent reduction. A (Fab').sub.2 fragment is a dimer of two Fab' fragments, held together by two disulfide bonds. An Fv fragment is defined as a genetically engineered fragment containing the variable region of a light chain and the variable region of a heavy chain expressed as two chains. A single chain antibody ("SCA") is a genetically engineered single chain molecule containing the variable region of a light chain and the variable region of a heavy chain, linked by a suitable, flexible polypeptide linker.
Ligands are intended to mean an agent that connects the cells to the microbeads independent on how the connection is formed or maintained. The cell could have several ligands that are connected or bound to the cell as well as the microbead could have several ligands coupled/bound on the surface. The ligand gives an affinity specific coupling between two objects, such as the cells and the microbeads. Examples of ligands includes antibodies (as defined above), monoclonal antibodies, affibodies, aptamers, other antigen specific molecules or fragments thereof specific for a subgroup of cells and microbeads.

**Methods and system**

This disclosure relates to micro scale method and systems for separating a subgroup of cells from a suspension, such as cells present in the blood.

Sample preparation

A suspension of cells, wherein said cells are a group of different cells, such as white blood cells are mixed with ligands (as defined above) specific for one group of cells and microbeads. The ligands are allowed to bind to one or more binding sites present on a subgroup of cells, such as epitopes on the cells, wherein one or more of the ligands may bind to one and the same cell. The microbeads are allowed to bind to the ligands either prior to the ligands binds to the cells or after.
In one embodiment the ligand is mixed with the microbeads and bound to the microbeads prior to that the ligand-microbead complex is further mixed with the cells. Thus the cells becomes affinity specific coupled to the ligand-microbead complex forming the cell-microbead complex.
The cells may be blood cells such as erythrocytes, platelets or leukocytes (such as: neutrophils, eosinophils, basophils, lymphocytes, monocytes, NK-cells and macrophages), as well as dendritic cells, stem cells/precursor cells, endothelial cells and epithelia cells. The beads may be polymer beads, magnetic beads, silica beads, metal beads. The size of the beads is smaller than the channel dimensions. Examples of antibodies are CD34 against hematopoietic stem cells, CD3, CD4 and CD8 against T-cells, CD19 and CD20 against B-cells, CD235a against erythrocytes, CD14 and CD33 against monocytes/macrophages, CD66b against granulocytes, CD11c and CD123 against dendritic cells, CD41, CD61 and CD62 against platelets, CD56 against NK-cells, CD236 against epithelial cells and CD146 against endothelial cells
**Acoustic step gradient liquid (ASGL)** Acoustic tailored liquid. Examples of ASGL includes Water, Ficoll, Percoll, Glycerol, PBS, Serum Albumine, Blood Plasma, NaCl as well as mixtures thereof. The density of ASGL being within 0.900 - 1.100 g/cm³, such as 1.000 - 1.077 g/cm³, 1.000-1.500 g/cm³, the compressibility of ASGL being within $2.10^{-10}$ - $1.10^{-9}$ Pa⁻¹, such as $5.10^{-10}$-$7.10^{-10}$ and the viscosity of the ASGL being within 0.5 to 50 mPa·s, such as 1-50 mPa·s, 5-50 mPa·s, 5-40 mPa·s, 0,5-10mPa·s and 0,5-5 mPa·s. The ASGL is important in this disclosure because it imposes a restriction on the objects, e.g. cells, when passing through the device. I.e. the physical properties of the cell-microbead complexes need to meet certain criteria regarding density and compressibility in order for them to be unlabeled cells.

Method

In one aspect this disclosure relates to a method, which comprises the steps of;

a) Forcing a suspension as defined above under sample preparation (51) via tubing (55) into at least one pre-alignment channel (15) by elevating the hydrostatic pressure $p_1$ in the container of the suspension (51) such that the pressure at the channel inlet (11) is higher than the pressure at any of the other fluidic connections (12, 13, and 14). Said pressure difference cause a laminar flow in the channel that may be varied from 1 μL/min to 2 mL/min. The elevated pressure may be established using a syringe pump, a peristaltic pump, or by regulating the gas pressure in a sealed chamber wherein the container of the fluid is placed.

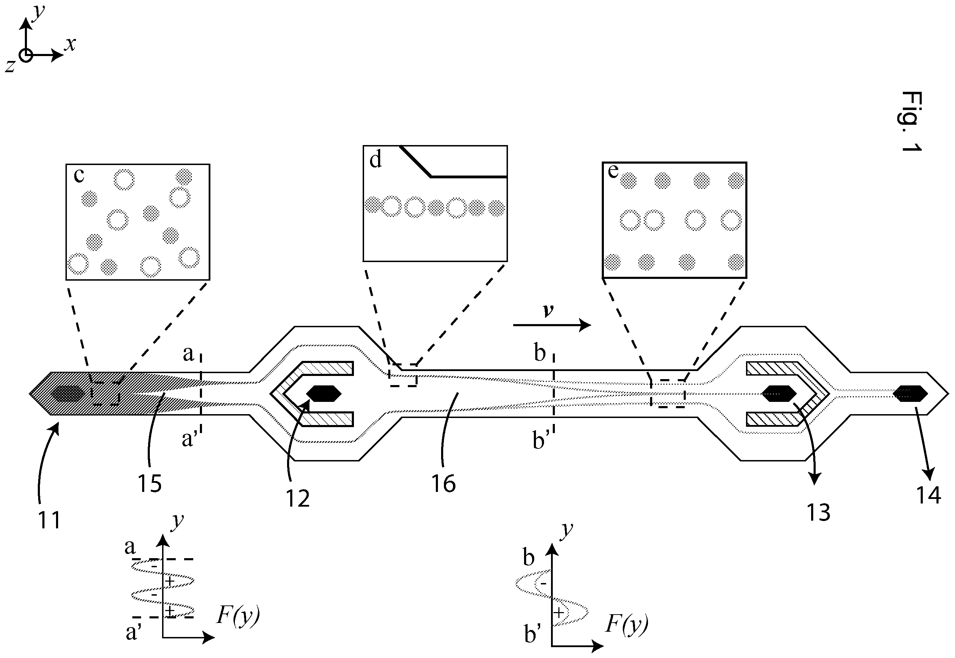

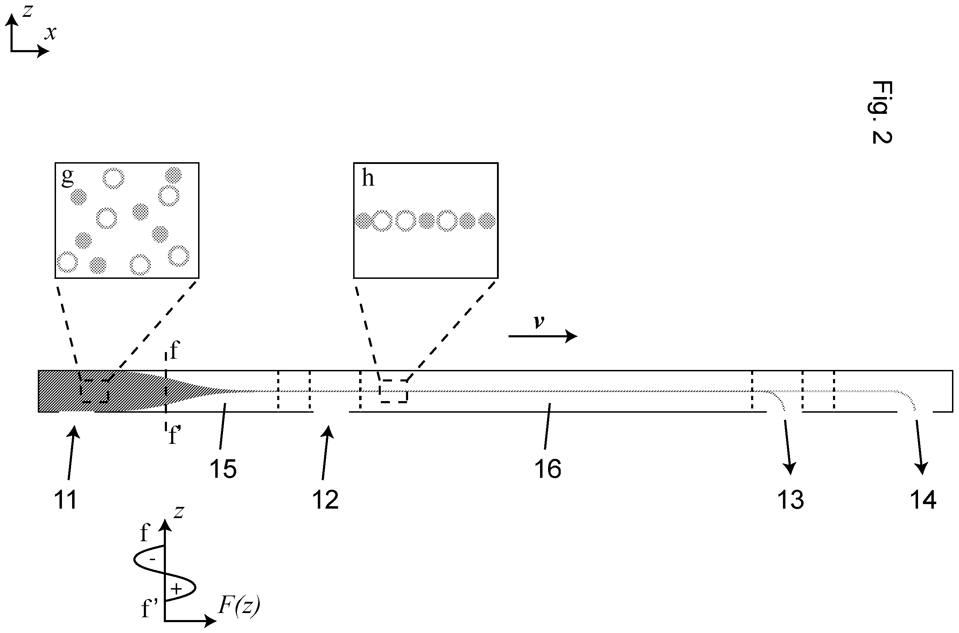

b) Subjecting the suspended cell and cell-microbead complexes (Fig. 1, inset c) in the suspension to a two-dimensional acoustic force potential (Fig. 1, a-a' and Fig. 2, f-f) directed perpendicular to the pre-alignment channel (15), so that all of the cells and cell-bead complexes are localized into one or more points in the transverse cross-section of the flow. The two-dimensional acoustic force potential stems from resonant modes of ultrasound inside the water filled channel caused by reflections in the interface between the acoustically liquid and the acoustically hard walls of the channel. By tuning the frequency of the ultrasound vibrations, modes can be excited that forces suspended cells and complexes away from the walls, floor and ceiling of the channel towards the nearest vibrational pressure node. The constant influx of suspension at (11) in combination with the two-dimensional acoustic force potential causes the cells and cell-bead complexes of the suspension to align in narrow bands just before exiting the pre-alignment channel (Fig. 1, inset d and Fig. 2, inset h).

The pre-alignment channel may have a width and/or height ranging from 75 μm to 800 μm, such as from 75 μm to 200 μm, or ranging from 200 μm to 375 μm, or ranging from 300 μm to 400 μm, or ranging from 400 μm to 700 μm, or ranging from 700 μm to 800 μm, or being 150 μm, 300 μm, 188 μm, 375 μm or 750 μm.

The width and height of the pre-alignment channel may be related such that the width w divided by an integer number n equals the height h divided by an integer number m. In this case a single frequency of vibration may be chosen to fulfill a resonance condition simultaneously for the height and width dimension, such that

$$ f = \frac{cn}{2w} = \frac{cm}{2h} $$

where c is the speed of sound in the suspending fluid. If the width and height of the channel are not related, the resonance condition may be controlled individually/selectively using separate frequencies of vibration for height and width, respectively. The frequency of vibration may vary in a range from 1 MHz to 10 MHz and is implicitly dictated by the dimensions of the channel as mentioned above and by choosing, either $n = 1$, $m = 2$, $c = 1500$ m/s. Other examples are from 1-5 MHz, such as from 2-5 MHz or being 2 MHz or 5 MHz.

One example of this two-dimensional acoustic force potential is when a liquid filled pre-alignment channel (15) of width $w_{15} = 300$ μm and height $h = 150$ μm for a vibrational frequency $f_{15} = 5$ MHz. For a stipulated speed of sound c in the suspension of 1500 m/s, the acoustic wavelength $\lambda_{15} = c/f_{15} = 300$ μm. Given that the channel only can support multiples of half wavelengths it is clear that the vibration can induce resonance in the channel both along the y-axis (Fig 1, a - a') and along the z-axis (Fig 2, f - f'). The combined yz-mode will have two vibrational pressure minima at $(y_1, z_1) = (w_{15}/4, h/2)$ and $(y_2, z_2) = (3w_{15}/4, h/2)$.

c) Forcing said acoustically pre-focused suspended objects and infusing ASGL via a central inlet (12) simultaneously into a second separation channel (16), wherein said mixture of cells and/or cell-bead complexes are forced to proximity of one or more walls of said second channel. The suspension is thus laminated along one or both sides of an acoustophoresis microchannel while particle free liquid occupies the remaining part of the channel. The relative volume flow rates $Q_2$ and $Q_1$, of the particle free liquid and the suspension, respectively, determine the lateral position ($y = w_1$) of the cells and/or particles, when entering the second channel (16). Increase of $Q_2$ leads to a decrease of $w_1$. The motive for doing so will be clear in paragraph (d and e).

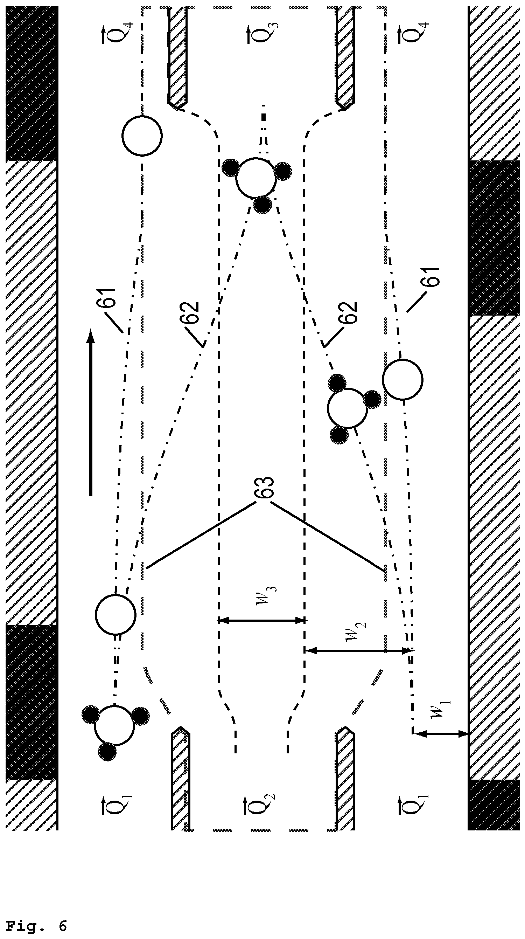

d) Subjecting said mixture of cells and complexes to a one dimensional acoustic force potential directed primarily along the *y*-axis of said separation channel, so that all of the objects move towards the vertical center plane of that channel. The one dimensional acoustic force potential stems from a resonant mode of ultrasound inside the water filled channel caused by reflections in the interface between the acoustically soft water and the acoustically hard walls of the channel. By tuning the frequency of the ultrasound vibrations, a half wavelength resonance mode can be excited that forces suspended objects away from the walls of the channel towards the vibrational pressure node in the channel center. The objects, that have previously been aligned by the 2-dimensional pre-alignment channel and are in proximity of the walls, move towards the vibrational pressure minima in the center of the channel. (Figs. 1 and 6)

When reaching the interface between their original suspending liquid and the ASGL the suspended cells and cell-bead complexes will not migrate further towards the center of the channel due the acoustic properties and/or the viscosity of the ASGL being different than those of the original suspending liquid. In order to propel a cell or cell-bead complex in an acoustic resonance the acoustic properties of the cell and cell-bead complexes and the suspending liquid must differ sufficiently. The composition of the ASGL can be chosen so that the transverse motion of non-complexed cells is kept at a minimum while cell-bead complexes can continue towards the vibrational pressure minima in the center of the channel. This is possible due to the fact that the beads have different acoustic properties compared to the cells. Bead-bound cells can thus be dragged by the acoustic force exerted on the beads into the center of the channel, which mediates separation based on the molecular expression levels on the surface of the cells. (Fig. 6)

Cells that express high levels of for example an epitope will statistically bind more beads than cells having low expression levels. This bias in the number of beads contained by each cell-bead complex will cause the complexes to migrate to the center at different rates. By dividing the flow into different outlets at the end of the channel the cell-bead complexes can be sorted into discrete fractions based on their surface marker expression level profile.

The separation channel may have a width ranging from 75 $\mu$m to 800 $\mu$m, such as from 75 $\mu$m to 200 $\mu$m, or ranging from 200 $\mu$m to 375 $\mu$m, or ranging from 300 $\mu$m to 400 $\mu$m, or ranging from 400 $\mu$m to 700 $\mu$m, or ranging from 700 $\mu$m to 800 $\mu$m, or being 150 $\mu$m, 300 $\mu$m, 188 $\mu$m, 375 $\mu$m or 750 $\mu$m.

The width may be chosen such that the frequency of vibration f is

$$f = \frac{cn}{2w}$$

where c is the speed of sound in the suspending fluid. The frequency of vibration may vary in a range from 1 MHz to 10 MHz and is implicitly dictated by the specified dimensions of the channel as mentioned above and by choosing n = 1 and c = 1500 m/s.

One example of this one-dimensional acoustic force potential can be a liquid filled separation channel (16) of width $w_{15}$ = 375 $\mu$m and height *h* = 150 $\mu$m for a frequency of vibration $f_{16}$ = 2 MHz. For a stipulated speed of sound c in the suspension of 1500 m /s, the acoustic wavelength $\lambda_{16}$ = $c/f_{16}$ = 750 $\mu$m. Given that the channel only can support multiples of half wavelengths it is clear that the vibration only can induce resonance in the channel along the *y*-axis (Fig 1, b - b '). This mode will have one vibrational pressure minima at *y* = $w_{16}/2$.

Each cell and cell-bead complexes move in the x-direction along the channel driven by the flow, while being forced towards the central vibrational node, in the *y*-direction, at a rate that is determined by the acoustomechanical properties, mass density and compressibility of the suspending medium and the cell and cell-bead complexes, respectively. The rate is also determined by the size of the cell and cell-bead complexes and the strength of the acoustic resonance. By tuning the amplitude of the acoustic resonance in the second channel, the paths of the cells and cell-bead complexes may be deflected so that cells and complexes of dissimilar acoustic mobility exit the channel at different locations along the y-axis.

Since all cells and cell-bead complexes are pre-aligned in the yz-plane when entering the separation channel, their individual trajectories, reflect their acoustophysical properties and size, to a high degree. In absence of the pre-alignment the trajectories of individual cells and/or particles would be strongly influenced by their initial position when entering the separation channel, which is indeed not an intrinsic property of the cell and cell-bead complexes.

e) Adjusting the volume flow rates in the inlets (Fig. 6, $Q_1$ and $Q_2$) and outlets $Q_3$ and $Q_4$ to maximize the travel distance ($w_2$) at the cost of processing time. By imposing a longer travel distance for a cells and complexes to reach the central outlet (13), the number of faulty cells and/or particles in that outlet will be reduced. Decreasing $Q_3$ relative to $Q_4$ will narrow down the hydrodynamic aperture ($w_3$) of the central outlet and cause an increased ($w_2$). Similarly, decreasing $Q_1$ relative to $Q_2$ will diminish ($w_1$). The increased selectivity must be balanced against the demands regarding processing time of a sample of some finite volume, which is related to $Q_1$, and the ability of the external fluidics to produce a stable enough flow, which is related to $Q_3$ relative any disturbance in $Q_4$.

System

In another aspect the present disclosure relates to a system for separating a subgroup of cells and cell-bead complexes from a mixture of different types of cells and cell-bead complexes in a suspension, comprising;

> i) a microchip having two inlets (11,12) and at least two outlets (13,14) and at least a first channel.
> ii) means to provide pressure, wherein said pressure forces said suspension through an inlet (11) and into at least a pre-alignment channel (15),
> iii) means to providing a two dimensional acoustic force (15),
> iv) means to introduce a ASGL (defined above) into said channel (12)
> v) means for providing a one dimensional acoustic force (16) and
> vi) means for collecting objects having the same size and/or mass density and/or compressibility from at least two outlets (13,14).

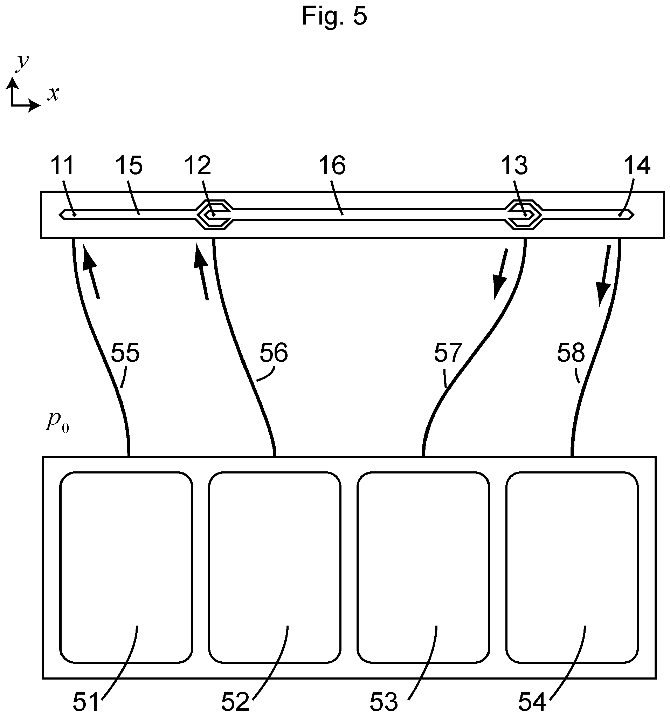

One example of a system is illustrated in the Figures 1-5. Fig. 1 and 2 shows how the suspension comprising cells and cell-bead complexes are forced into the system (11). The suspension with the cells and cell-bead complexes (Fig. 1, insert c) is then transported through the pre-alignment channel (15) and exposed to a first two-dimensional acoustic force potential acting primarily in the *yz*-plane (Fig. 1, a-a' and Fig. 2, f-f, respectively) and the cells and/or cell-bead complexes are focused into two spatially separated flow streams. The two streams are then guided into two separate channels and ASGL is introduced into the system through (12). The ASGL forces the two pre-aligned streams of cells and cell-bead complexes out to positions proximal to the walls of the channel (Fig. 1, inset d). The cells and cell-bead complexes are then exposed to a one-dimensional acoustic force (Fig 1, b-b') (16), which separates cells and the cell-bead complexes from each other (Fig 1, inset e). Cell-bead complexes are collected at (13) while non-complexed cells are collected at (14). The same can be seen in figures 5, which is a simplified picture of what happens in the system upon use.

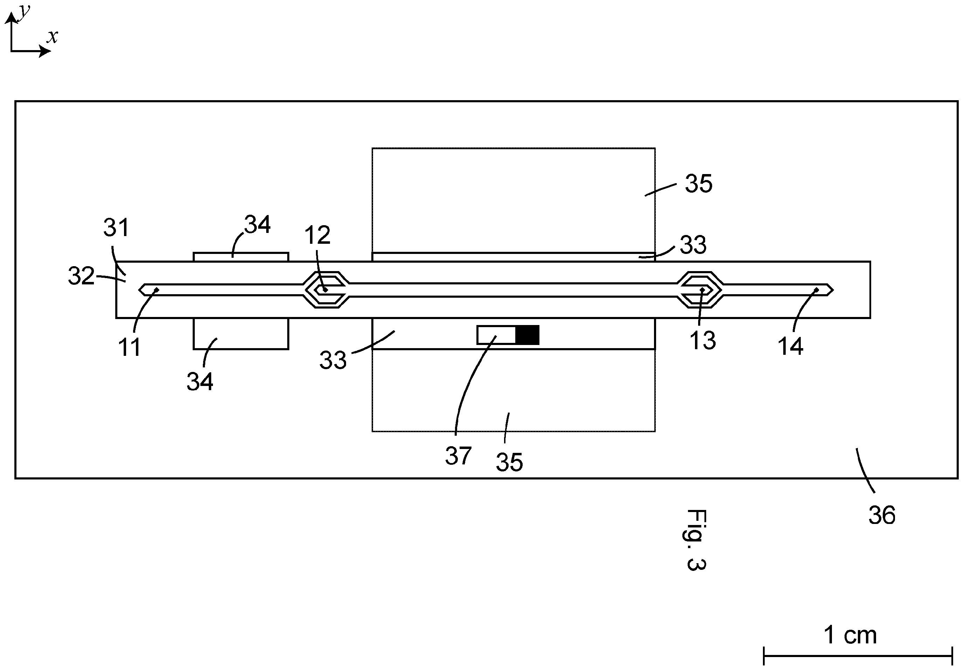

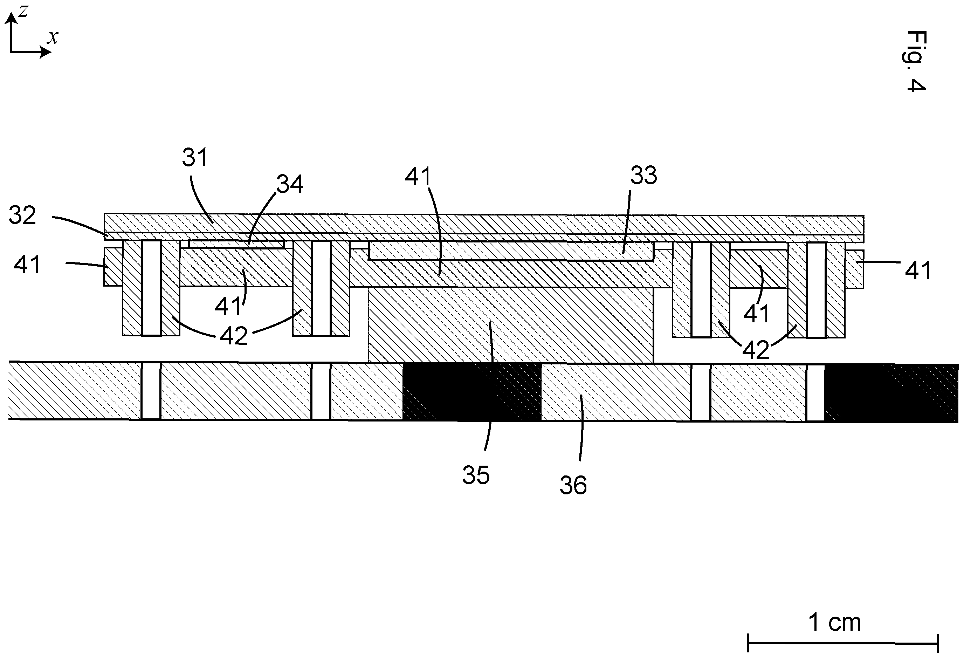

In one embodiment the microchip may be mounted as shown in figure 3 and 4, which are two views of the same microchip.

In figure 3 there is an inlet (11) for the cells and cell-bead complexes in a suspension to enter the microchip. There is an inlet for the ASGL (12) and there are at least two outlets, one central (13) and one side outlet (14), where the cells and cell-bead complexes are collected.

Over the microchip with the channel there is a glass cover/ceiling to seal the microchannel (31). There is also silicon substrate (32) wherein the channel segments are etched. There is a transducer (33) for actuation of ultrasound in separation chamber. There is also a second transducer 2 present (34) for actuation of ultrasound for two-dimensional pre-alignment. In this embodiment there is also a Peltier element (35) present to be able to control the temperature in the device. In addition, is an aluminum microscope mounting plate / heat sink (36) present, to absorb excess heat from the Peltier element. A PT-100 thermo resistive temperature sensor (37) is used to monitor temperature for a feedback control loop.

In figure 4 an aluminum plate (41) is used for even distribution of heat in the device. There are inlet connectors (42), pieces of silicone tubing, the other references are as shown in figure 3 and explained above.

In figure 5, being a simplified overview of the system in operation. There is a pressure chamber for input cell and/or particle suspension (51), wherein the cells and the cell-bead complexes are forced into the microchip. There is one pressure chamber for ASGL(52), which forces the solution into the microchip. There are also two pressure chambers for the central outlet (53) and the sides' outlet (54). There are also four containers present in the chambers, one for the cell and our particle suspension (51), one for the ASGL (52), that may be any solution such as those mentioned above, one container for the central outlet (53) and one for the sides' outlet (54). There are also a number of tubings (55, 56, 57 and 58) that allows the transfer of the liquids from the chambers (51) and (52) and the two chambers (53) and (54).

In one example the acoustic separation system is operated under iso-thermal conditions since thermal fluctuation may in some embodiments severely influences the acoustic properties of the microchannel acoustic resonators and hence the separation outcome. This may be realized by mounting the microfluidic acoustic separation chip on a Peltier-element that is computer controlled via a temperature sensor feedback at the separation chip vicinity. Thereby the temperature over the whole microchip is maintained at the same level. If the temperature is too high it might influence the cells and/or the particles. One example being when cells are to be separated that later are to be transferred into a mammal, such as a human being. If the cells are exposed to too high temperatures they get stressed or may die and cannot be transferred back to the subject in need of those cells.

The size of the microchannel constitutes an upper limit of the size of the cells and/or particles to be separated. The cells and/or particles to be separated may vary in shape and size ranging from 1 $\mu$m to 50 $\mu$m, such as 1-5 $\mu$m, 1-25 $\mu$m, 5-50 $\mu$m, 5-40 $\mu$m, 5-30 $\mu$m, 5-25 $\mu$m, 8-25 $\mu$m, or 8, 9, 10, 11, 12, 13, 14, 15, 16, 17, 18, 19 or 20 $\mu$m, or from 10-20 $\mu$m or 10-15 $\mu$m. The cells or particles may have a volume ranging from 0.0005 to 70 x $10^{-15}$ m$^3$, such as 0.0005-0.003 x $10^{-15}$ m$^3$, 0.0005-0.07 x $10^{-15}$ m$^3$, 0.0005-8 x $10^{-15}$ m$^3$, 0.05-0.10 x $10^{-15}$ m$^3$, 0.07-70 x $10^{-15}$ m$^3$, 0.07-35 x $10^{-15}$ m$^3$, 0.07-14 x $10^{-15}$ m$^3$, 0.07-8 x $10^{-15}$ m$^3$, 0.25-6 x $10^{-15}$ m$^3$ 0.3-8 x $10^{-15}$ m$^3$, 0.07-35 x $10^{-15}$ m$^3$ or 0.5-15 x $10^{-15}$ m$^3$.

Following examples are intended to illustrate but not to limit the invention in any manner, shape, or form, either explicitly or implicitly.

EXAMPLES

**Experimental setting**

A microchannel structure and holes for inlets and outlets was KOH etched in a <100> silicon wafer of thickness 350 $\mu$m and cut to the dimensions 40 mm by 3 mm (32). A piece of borosilica glass (31) (40 mm by 3 mm by 1 mm) was anodically bonded to seal the channel. Inlets and outlets comprises of pieces of silicone tubing (42) which are glued to the backside of the chip to connect tubing for external fluidics.

All parts of the chip assembly were glued together. From bottom and up sandwiched together: an aluminum mounting plate also serving as heat sink, a Peltier element (35) (15 mm by 15 mm), an aluminum bar (41) (40 mm by 3 mm by 2 mm) with holes drilled to match the radius of the silicone inlet/outlet tubing, two piezoceramic actuators, one ~5 MHz transducer (34) (5 mm by 5 mm) positioned below the pre-alignment channel, and one ~2 MHz transducer (33) (15 mm by 5 mm) placed under the separation channel, and the acoustophoresis chip, see Fig. 3 and 4. A Pt100 thermo-resistive element for temperature measurement, was glued onto the 2 MHz piezoceramic actuator alongside the acoustophoresis chip, see Fig. 3.

The piezoceramic transducers are driven by two signal-generators equipped with signal power amplifiers. The acoustic resonances can be controlled by tuning the frequency and voltage over the transducers.

Cells/microparticles in suspension enter a first pre-focusing channel (15) at a flow rate of 50 or 70 $\mu$L min$^{-1}$. A 5 MHz resonance in the yz-plane focus particles in two narrow bands, see Fig 1 and 2. Clean buffer medium enters through (12) at a volume flow rate of 450 or 490 $\mu$L/min and divides the pre-aligned particles further so that the stream of particles are pushed close to the walls of a separation channel (16). A 1.94 MHz resonance along the y-coordinate only focuses the particles towards the channels vertical center plane. At the end of the separation channel the flow is split up in a trifurcation outlet (13 and 14). The central outlet (13) volume flow is set to 150 or 280 $\mu$L/min and the volume flow rate in the combined side's outlet (14) was 250 or 280 $\mu$L/min.

**Example 1,** Separation of T cells from mononuclear cells

*Sample preparation.* Peripheral blood progenitor cell samples were obtained from consenting healthy stem cell donors and patients undergoing leukapheresis. Mononuclear cells were isolated by density centrifugation and cell were incubated with super-paramagnetic polystyrene beads (Dynabeads, Invitrogen, Dynal CD4 positive, 4.5 $\mu$m in diameter) according to the manufacturer's instructions For flow cytometry (FACS) analysis and functional studies the beads were released from the cells using DETACHaBEAD solution.

*Flow system setup.* The volume flow in the system is controlled by three 10 mL glass syringes (1010 TLL, Hamilton Bonaduz AG, Bonaduz, Switzerland) which are mounted on a syringe pump (neMESYS, Cetoni GmbH, Germany). Two syringes on the outlets are set in withdrawal mode for maintaining a volume flow $Q_3$ = 30 $\mu$L/min in the central outlet and $Q_4$ = 60 $\mu$l/min in the combined side's outlet, respectively (see Fig. 6 for definition of $Q_i$). The flow rates of these two syringes dictate the total volume flow ($Q_{total}$ = 90 $\mu$L/min) rate in the main channel. The third syringe, infuses cell-free liquid in the central inlet to the channel at a volume flow rate corresponding to 67% of the total flow rate ($Q_2$ = 60 $\mu$L/min). For separation of bead bound cells from the mononuclear cell mixture, the liquid in the third syringe has to be more dense than the cell suspension. For the density step was undiluted Ficoll (Ficoll Histopaque, 1.077g/cm3) used. This density step enable separation of the bead labeled cell from the cell mixture. If the denser liquid is not used, all cells would act similar in the sound field and no separation will be possible. The cell mixture was drawn into the side's inlet from the bottom of a test tube, at atmospheric pressure, at a flow rate of 33% of the total volume flow rate ($Q_1$ = 30 $\mu$L/min) dictated by the net flux created by the three syringes.

*Analysis of acquired outlet fractions.* After acoustophoresis the cell and bead suspensions in the central respectively side outlet fractions were treated with DETACHaBEAD solution to break the bead bond to the cells. Magnetic beads were then removed with a magnet. Cells were then centrifuged, supernatant was removed, cell pellet labeled with CD4 fluorescent antibodies that target T-cells, washed and enumerated by flow cytometry (FACS Canto II flow cytometer and the FACSDiva software, BD Biosciences).

*Acoustophoresis cell separation procedure.* The ultrasound actuation frequency was adjusted to approximately 4.68 MHz in the first two-dimensional pre-focusing channel and in the second, separation channel, a fundamental resonance of 1.92MHz was used. The driving voltage to the pre-focusing transducer was approximately 5Vpp (2-7V$_{pp}$) and to the main separation transducer approximately 3V$_{pp}$ (1-5V$_{pp}$). Because the use of ASGL in the center inlet, the mononuclear cells will not move into the center part of the chip but the complex will. The amplitude of the standing wave is set so that the complex barely just enter the center outlet. By doing this careful balance, mononuclear cells are prevented to slip into the center outlet stream. The amount of T-cells in the center fraction was enriched to 90 $\pm$7 %, compared to approximately 20% in the original sample.

## Claims

1. A micro scale method for separating cell-bead complexes, said cell-bead complexes comprising cells, ligands and beads, from a mixture of different types of cells in a suspension, said suspension further comprising a suspending liquid, comprising the steps of;

   i) subjecting said suspension to pressure, wherein said pressure forces said suspension into at least one inlet (11) and into at least one channel present on a microfluidic chip,
   ii) forcing said suspension through the side branches of a trifurcated inlet while forcing cell free acoustic step gradient liquid, said acoustic step gradient liquid having physical properties which are different from the physical properties of the suspending liquid, through the central branch of said trifurcated inlet (12) simultaneously into at least one separation channel, wherein said suspension is pushed by hydrodynamic forces towards the walls of the separation channel,
   iii) subjecting said mixture of different cells and said cell-bead complexes to a one dimensional acoustic force directed perpendicular to the length direction towards the center of the separation channel (16) and
   iv) collecting said cell-bead complexes at the end of the separation channel present on the microfluidic chip through at least one first outlet (13),
   v) collecting said mixture of different types of cells at the end of the separation channel present on the microfluidic chip through at least one second outlet (14).

2. The method according to claim 1, wherein said ligand is antibodies, monoclonal antibodies, affibodies, aptamers, single chain antibodies, antigen specific molecules or fragments thereof.

3. The method according to claim 2, wherein said cells are selected from the group consisting erythrocytes, platelets or leukocytes dendritic cells, stem cells/precursor cells, endothelial cells and epithelia cells.

4. The method according to any of preceding claims, wherein said beads are selected from the group consisting of the beads may be polymer beads, superparamagnetic beads, silica beads, metal beads.

5. The method according to any preceding claim, wherein said acoustic step gradient liquid has a density between 0.900 - 1.100 g/cm$^3$ or a density between 1.000 - 1.500 g/cm$^3$.

6. The method according to any of preceding claims, further comprising the step of subjecting said suspension to a two dimensional acoustic force directed perpendicular to the length direction of the channel (15) after subjecting said suspension to said pressure, and prior to forcing said suspension through said trifurcated inlet.

7. The method according to claim 6, wherein the two dimensional acoustic force consists of 1/2 wavelength and 1 wavelength perpendicular to each other.

8. The method according to any of the preceding claim, wherein said one dimensional acoustic force in step iv) consists of 1/2 wavelength.

9. The method according to claim 6, wherein said two dimensional acoustic force is caused by a frequency of from 1 to 10 MHz.

10. The method according to any of the preceding claims, wherein said method is maintained at a constant temperature.

11. The method according to any of the preceding claims, wherein the height or the width of channel is from 75 um to 800 um.

12. The method according to any of the preceding claims, further comprising the step of, prior to subjecting said suspension to said pressure, mixing said mixture of different types of cells in said suspending liquid with said ligands and beads to obtain said suspension.

## Patentansprüche

1. Verfahren im Mikromaßstab zur Trennung von Zellkügelchenkomplexen, wobei die Zellkügelchenkomplexe Zellen, Liganden und Kügelchen umfassen, von einem Gemisch aus unterschiedlichen Arten von Zellen in einer Suspension, wobei die Suspension ferner eine Suspensionsflüssigkeit umfasst, die folgenden Schritte umfassend:

   i) Aussetzen der Suspension gegenüber Druck, wobei der Druck die Suspension in zumindest einen Einlass (11) und in zumindest einen Kanal drängt, der an einem mikrofluidischen Chip vorhanden ist,
   ii) Drängen der Suspension durch die Seitenzweige eines dreigeteilten Einlasses, während zellfreie Akustikstufengradientenflüssigkeit, wobei die Akustikstufengradientenflüssigkeit physische Eigenschaften aufweist, die sich von den physischen Eigenschaften der Suspensionsflüssigkeit unterscheiden, durch den zentralen Zweig des dreigeteilten Einlasses (12) simultan in zumindest einen Trennkanal gedrängt wird, wobei die Suspension durch hydrodynamische Kräfte in Richtung der Wände des Trennkanals gedrückt wird,

iii) Aussetzen des Gemischs aus unterschiedlichen Zellen und der Zellkügelchenkomplexe gegenüber einer eindimensionalen akustischen Kraft, die senkrecht zu der Längsrichtung in Richtung der Mitte des Trennkanals (16) gerichtet ist, und

iv) Sammeln der Zellkügelchenkomplexe am Ende des Trennkanals, der an dem mikrofluidischen Chip vorhanden ist, durch zumindest einen ersten Auslass (13),

v) Sammeln des Gemischs aus unterschiedlichen Arten von Zellen am Ende des Trennkanals, der an dem mikrofluidischen Chip vorhanden ist, durch zumindest einen zweiten Auslass (14).

2. Verfahren nach Anspruch 1, wobei der Ligand Antikörper, monoklonale Antikörper, Affibodys, Aptamere, Einzelkettenantikörper, antigenspezifische Moleküle oder Fragmente davon ist.

3. Verfahren nach Anspruch 2, wobei die Zellen aus der Gruppe ausgewählt sind, die aus Erythrozyten, dendritischen Zellen von Thrombozyten oder Leukozyten, Stammzellen/Vorläuferzellen, endothelialen Zellen und Epithelzellen besteht.

4. Verfahren nach einem der vorhergehenden Ansprüche, wobei die Kügelchen aus der Gruppe ausgewählt sind, die aus den Kügelchen besteht, die Polymerkügelchen, superparamagnetische Kügelchen, Kieselsäurekügelchen, Metallkügelchen sein können.

5. Verfahren nach einem vorhergehenden Anspruch, wobei die Akustikstufengradientenflüssigkeit eine Dichte zwischen 0,900-1.100 g/cm$^3$ oder eine Dichte zwischen 1.000-1.500 g/cm$^3$ aufweist.

6. Verfahren nach einem der vorhergehenden Ansprüche, ferner umfassend den Schritt des Aussetzens der Suspension gegenüber einer zweidimensionalen akustischen Kraft, die senkrecht zu der Längsrichtung des Kanals (15) gerichtet ist, nachdem die Suspension gegenüber dem Druck ausgesetzt wird und bevor die Suspension durch den dreigeteilten Einlass gedrängt wird.

7. Verfahren nach Anspruch 6, wobei die zweidimensionale akustische Kraft aus 1/2 Wellenlänge und 1 Wellenlänge besteht, die senkrecht zueinander sind.

8. Verfahren nach einem vorhergehenden Anspruch, wobei die eindimensionale akustische Kraft in Schritt iv) aus 1/2 Wellenlänge besteht.

9. Verfahren nach Anspruch 6, wobei die zweidimensionale akustische Kraft durch eine Frequenz von 1 bis 10 MHz bewirkt wird.

10. Verfahren nach einem der vorhergehenden Ansprüche, wobei das Verfahren bei einer konstanten Temperatur gehalten wird.

11. Verfahren nach einem der vorhergehenden Ansprüche, wobei die Höhe oder die Breite des Kanals 75 um bis 800 um beträgt.

12. Verfahren nach einem der vorhergehenden Ansprüche, ferner umfassend den Schritt des Mischens des Gemischs aus unterschiedlichen Arten von Zellen in der Suspensionsflüssigkeit mit den Liganden und Kügelchen, um die Suspension zu erhalten, bevor die Suspension gegenüber dem Druck ausgesetzt wird.

## Revendications

1. Procédé à l'échelle microscopique de séparation de complexes cellules-billes, lesdits complexes cellules-billes comprenant des cellules, des ligands et des billes, d'un mélange de différents types de cellules en suspension, ladite suspension comprenant en outre un liquide de suspension, comprenant les étapes :

i) de soumission de ladite suspension à une pression, dans lequel ladite pression force ladite suspension dans au moins une entrée (11) et dans au moins un canal présent sur une puce microfluidique,

ii) de forçage de ladite suspension à travers les branches latérales d'une entrée trifurquée tout en forçant un liquide à gradient discontinu acoustique sans cellules, ledit liquide à gradient discontinu acoustique ayant des propriétés physiques différentes des propriétés physiques du liquide de suspension, à travers la branche centrale de ladite entrée trifurquée (12) simultanément dans au moins un canal de séparation, dans lequel ladite suspension est poussée par des forces hydrodynamiques vers les parois du canal de séparation,

iii) de soumission dudit mélange de différentes cellules et desdits complexes cellules-billes à une force acoustique unidimensionnelle dirigée perpendiculairement à la direction longitudinale vers le centre du canal de séparation (16) et

iv) de collecte desdits complexes cellules-billes au niveau de l'extrémité du canal de séparation présent sur la puce microfluidique à travers au moins une première sortie (13),

v) de collecte dudit mélange de différents types de cellules au niveau de l'extrémité du canal de séparation présent sur la puce microfluidique à travers au moins une seconde sortie (14).

2. Procédé selon la revendication 1, dans lequel ledit ligand est des anticorps, des anticorps monoclonaux, des afficorps, des aptamères, des anticorps monocaténaires, des molécules spécifiques d'un antigène ou des fragments de ceux-ci.

3. Procédé selon la revendication 2, dans lequel lesdites cellules sont choisies dans le groupe constitué des cellules dendritiques érythrocytaires, plaquettaires ou leucocytaires, des cellules souches/précurseurs, des cellules endothéliales et des cellules épithéliales.

4. Procédé selon l'une quelconque des revendications précédentes, dans lequel lesdites billes sont choisies dans le groupe constitué des billes pouvant être des billes de polymère, des billes superparamagnétiques, des billes de silice, des billes métalliques.

5. Procédé selon une quelconque revendication précédente, dans lequel ledit liquide à gradient discontinu acoustique a une densité comprise entre 0,900 et 1 100 g/cm$^3$ ou une densité comprise entre 1 000 et 1 500 g/cm$^3$.

6. Procédé selon l'une quelconque des revendications précédentes, comprenant en outre l'étape de soumission de ladite suspension à une force acoustique bidimensionnelle dirigée perpendiculairement à la direction longitudinale du canal (15) après la soumission de ladite suspension à ladite pression, et avant le forçage de ladite suspension à travers ladite entrée trifurquée.

7. Procédé selon la revendication 6, dans lequel la force acoustique bidimensionnelle est constituée d'une demi-longueur d'onde et d'une longueur d'onde perpendiculaires l'une par rapport à l'autre.

8. Procédé selon une quelconque revendication précédente, dans lequel ladite force acoustique unidimensionnelle à l'étape iv) comprend une demi-longueur d'onde.

9. Procédé selon la revendication 6, dans lequel ladite force acoustique bidimensionnelle est provoquée par une fréquence de 1 à 10 MHz.

10. Procédé selon l'une quelconque des revendications précédentes, dans lequel ledit procédé est maintenu à une température constante.

11. Procédé selon l'une quelconque des revendications précédentes, dans lequel la hauteur ou la largeur du canal est comprise entre 75 um et 800 um.

12. Procédé selon l'une quelconque des revendications précédentes, comprenant en outre l'étape, avant la soumission de ladite suspension à ladite pression, de mélange dudit mélange de différents types de cellules dans ledit liquide de suspension avec lesdits ligands et billes pour obtenir ladite suspension.

Fig. 1

EP 2 897 709 B1

Fig. 2

EP 2 897 709 B1

Fig. 3

Fig. 4

EP 2 897 709 B1

1 cm

# Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007128795 A

**Non-patent literature cited in the description**

- **R. S. MOLDAY ; S. P. S. YEN ; A. REMBAUM.** *Nature,* 1977, vol. 268, 437-438
- **R. S. MOLDAY ; L. L. MOLDAY.** *Febs Letters,* 1984, vol. 170, 232-238
- **S. MILTENYI ; W. MULLER ; W. WEICHEL ; A. RADBRUCH.** *Cytometry,* 1990, vol. 11, 231-238